# EUROPÄISCHE PATENTSCHRIFT

(11) **EP 2 844 635 B1**
(45) Veröffentlichungstag und Bekanntmachung des Hinweises auf die Patenterteilung: **15.03.2017**
(21) Anmeldenummer: 13720351.9
(22) Anmeldetag: 29.04.2013
(51) Int. Cl.: C07C 209/84, C07C 211/46, C07C 211/50

(54) **VERFAHREN ZUR BEHANDLUNG EINES EIN AROMATISCHES AMIN UMFASSENDEN STOFFGEMISCHES, INSBESONDERE VON ROH-ANILIN**
METHOD FOR TREATING A MATERIAL MIXTURE COMPRISING AN AROMATIC AMINE, IN PARTICULAR RAW ANILINE
PROCÉDÉ DE TRAITEMENT D'UN MÉLANGE DE MATIÈRE COMPRENANT UNE AMINE AROMATIQUE, EN PARTICULIER D'ANILINE BRUTE

(30) Priorität: 04.05.2012 EP 12166832
(43) Veröffentlichungstag der Anmeldung: 11.03.2015
(73) Patentinhaber: Covestro Deutschland AG, 51373 Leverkusen (DE)
(72) Erfinder: MERKEL, Michael, 40223 Düsseldorf (DE); KNAUF, Thomas, 41542 Dormagen (DE); PETERS, Cliff Andre, 25724 Schmedeswurth (DE); SCHMIDT, Thorsten, 25704 Nindorf (DE)
(74) Vertreter: Levpat
(86) Internationale Anmeldenummer: PCT/EP2013/058898
(87) Internationale Veröffentlichungsnummer: WO 2013/164308

(56) Entgegenhaltungen:
- EP-A1- 0 696 574
- EP-A1- 0 794 170
- EP-A1- 1 005 888

## Beschreibung

Die vorliegende Erfindung betrifft ein Verfahren zur Behandlung eines ein aromatisches Amin umfassenden Stoffgemisches, wobei das Stoffgemisch ein erstes Stoffgemisch ist und ein aromatisches Amin und Verbindungen mit einem höheren Siedepunkt als das aromatische Amin umfasst, mit dem Schritt: I) Destillatives Auftrennen des ersten Stoffgemisches in einer ersten Destillationseinheit unter zumindest teilweiser Abtrennung des aromatischen Amins, wobei weiterhin ein erstes Sumpfprodukt erhalten wird und dieses erste Sumpfprodukt aus der ersten Destillationseinheit ausgetragen wird. Dieses Sumpfprodukt kann dann mit im Produktionsbetrieb ohnehin anfallenden Abfällen verdünnt werden, wodurch der Verlust an aromatischen Aminen im Sumpfprodukt verringert werden kann. Das aromatische Amin ist erfindungsgemäß Anilin oder 2,4-Diaminotoluol, bevorzugt Anilin.

Aromatische Amine sind wichtige Zwischenprodukte, die preiswert und in großen Mengen hergestellt werden müssen. Daher werden Produktionsanlagen für aromatische Amine in der Regel für sehr große Kapazitäten errichtet. Die hohe Produktivität dieser Anlagen ist durch sehr lange Reaktionszyklen und den störungsfreien Ablauf zwischen den An- und Abfahrvorgängen der Hydrierung zur Regeneration der eingesetzten Hydrierkatalysatoren gewährleistet.

Das Haupteinsatzgebiet für 2,4-Diaminotoluol ist die Herstellung von Toluylendiisocyanat (TDI). Es wird großtechnisch durch die Hydrierung von 2,4-Dinitrotoluol hergestellt.

Das Haupteinsatzgebiet für Anilin ist die Herstellung von Methylendiphenyldiamin (MDA), das zur Herstellung von Methylendiphenyldiisocyanat (MDI) dient. Anilin wird im großtechnischen Maßstab in der Regel durch katalytische Hydrierung von Nitrobenzol mit Wasserstoff hergestellt. Besonders bevorzugt sind Reaktionsführungen wie in EP 0 944 578 A2 (isotherme Fahrweise) und in EP 0 696 574 B1, EP 0 696 573 B1 und EP 1 882 681 A1 (adiabate Fahrweise) beschrieben. Die Herstellung von MDA ist in zahlreichen Patenten und Publikationen beschrieben (siehe z.B. H.J. Twitchett, Chem. Soc. Rev. 3(2), 209 (1974), M.V. Moore in: Kirk-Othmer Encycl. Chem. Technol., 3rd. Ed., New York, 2, 338-348 (1978)).

Den beschriebenen isothermen Verfahren zur Herstellung von Anilin ist gemein, dass der Ausgangsstoff Nitrobenzol bei erhöhter Temperatur im Wasserstoff-Strom verdampft wird.

Die Reaktionsführung erfolgt im Allgemeinen so, dass das gasförmige Nitrobenzol / Wasserstoff-Gemisch in den Hydrierreaktor geleitet und hier am Festbett-Katalysator, gegebenenfalls mit anschließendem Nachreaktor, bei erhöhter Temperatur und Normaldruck umgesetzt wird.

Die freiwerdende Reaktionswärme wird über einen Wärmetauscher aus dem Reaktor abgeführt und in der Regel zur Erzeugung von Heizdampf genutzt.

Die Reaktionsprodukte Anilin und Wasser verlassen den Reaktor gasförmig und werden aus dem Wasserstoff-Strom über eine mehrstufige Kondensation auskondensiert. Der überschüssige Wasserstoff wird im Kreis gerührt, durch Frisch-Wasserstoff ergänzt und wieder gemeinsam mit Nitrobenzol verdampft und als Gemisch in den Hydrierreaktor geleitet.

Durch die Kreisfahrweise belädt sich der Wasserstoff mit gasförmigen Verunreinigungen. Zur Ausschleusung dieser Verunreinigungen wird dem Wasserstoff-Kreislauf ein Teilstrom entnommen und in der thermischen Abluftreinigung verbrannt.

Die auskondensierten Reaktionsprodukte trennen sich in eine organische Phase (Rohanilin) und eine wässrige Phase (Anilin-Wasser), die separat weiterverarbeitet werden. Das Rohanilin enthält noch Wasser und organische Nebenprodukte in gelöster Form, die destillativ abgetrennt werden.

Zunächst werden in einer Kolonne über Kopf die leichtsiedenden Nebenkomponenten (z. B. Cyclohexylamin, Cyclohexanon, Benzol) und im Seitenstrom das Wasser als Anilin-Wasser-Azeotrop abdestilliert. Der Seitenstrom ist zweiphasig und wird in die o. g. Phasentrennung zurückgeführt.

Die leichtsiedenden Nebenkomponenten, die auch noch Anilin enthalten, werden am Kopf der Kolonne abgezogen und können entweder direkt in einer Verbrennungsanlage verwertet werden oder zunächst kondensiert und später zusammen mit anderen Rückständen verbrannt werden.

Das Sumpfprodukt (Anilin+Schwersieder) wird in einer zweiten Destillationskolonne von den schwersiedenden Nebenprodukten (z. B. N-Cyclohexylanilin, N,N-Diphenylamin, Phenol) befreit. Über Kopf destilliert das Reinanilin ab. Im Sumpf reichern sich die Schwersieder an, die in einer dritten Destillationskolonne (Rückstandskolonne) weiter eingeengt werden.

Am Kopf der Rückstandskolonne wird Anilin zurückgewonnen und zusammen mit dem Sumpfprodukt der ersten Kolonne zurück in die zweite Kolonne gefahren. Der Sumpf der Rückstandskolonne wird in einen Rückstandsbehälter überführt. Dabei dient ein Restgehalt an Anilin als Lösungsmittel dazu, die Pumpfähigkeit des Rückstandes zu erhalten. Außerdem wird der Rückstand bei erhöhter Temperatur gelagert, um Niederschläge oder zu hohe Viskositäten zu vermeiden. Aus dem Rückstandsbehälter werden die Schwersieder zusammen mit dem zur Verdünnung dienenden Restanilin einer Verbrennung zugeführt.

Das in der Phasentrennung separierte Wasser wird destillativ vom gelösten Anilin befreit und als Abwasser in die biologische Abwasseraufbereitung des Werkes gegeben. Das Anilin destilliert als Azeotrop mit Wasser ab und wird in die o. g. Phasentrennung zurückgeführt.

Alle Stufen des Verfahrens zur Herstellung von Anilin werden in kontinuierlicher Betriebsweise durchgeführt.

Die Güte eines Verfahrens zur Hydrierung aromatischer Nitroverbindungen ist einerseits definiert durch die Qualität des Produktes. Andererseits ist die Güte eines Hydrierverfahrens dadurch definiert, dass der gesamte Prozess kontinuierlich ohne nennenswerte Produktionsausfälle betrieben werden kann. In der Regel spricht man von einem reibungslosen Ablauf im Hydrierzyklus, beim Abfahren der Hydrierung, der Regeneration des Hydrierkatalysators und bei dem wieder Anfahren des Hydrierprozesses.

Da alle Stufen des Verfahrens zur Herstellung von Anilin während des Hydrierzyklus in kontinuierlicher Betriebsweise durchgeführt werden, gilt es auch die Aufarbeitung von Rohanilin störungsfrei zu bedienen. Zu guter Letzt ist es auch wichtig eine hohe Ausbeute am gewünschten Produkt zu erzielen, dies heißt, Nebenprodukte in der Reaktion zu vermeiden und Produktionsverluste in der Anlage zu minimieren. Solche Verluste treten zum Beispiel in der Destillation des Produkts auf, da eine Aufkonzentrierung der schwersiedenden Nebenkomponenten im Sumpf nur bis zu einem gewissen Grad möglich ist, ohne dass der Rückstand sich verfestigt oder Teile davon ausfallen und unerwünschte Niederschläge bilden. Deshalb wird mit den Nebenkomponenten stets ein nennenswerter Anteil an Produkt verbrannt.

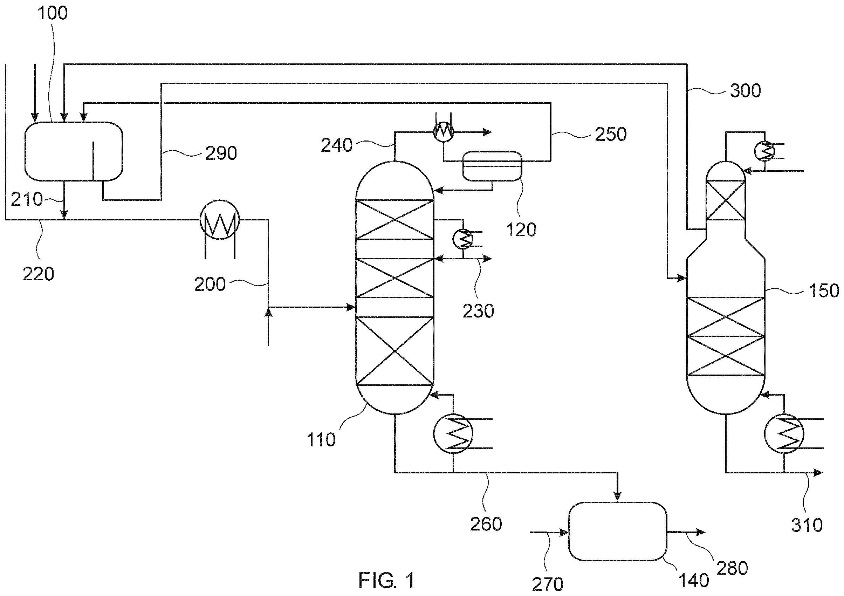

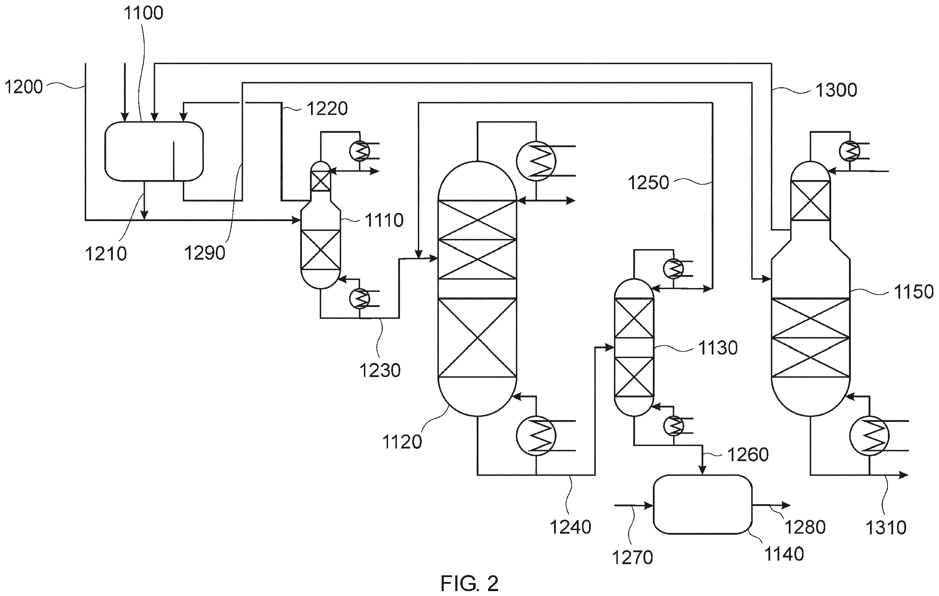

EP 0 794 170 A1 offenbart ein Verfahren zur Schwersiederabtrennung bei der Diaminotoluol-Herstellung. Das rohe, entwässerte Diaminotoluol wird in eine Destillationseinheit gerührt, die eine Packungskolonne mit Umlaufverdampfer für den Kolonnensumpf umfasst. In einer Ausführungsform (Fig. 1) ist der Packungskolonne ein Fallfilmverdampfer, in welchen der Sumpfaustrag der Packungskolonne geleitet wird, nachgeschaltet. In einer anderen Ausführungsform (Fig. 2) ist der Packungskolonne ein Fallfilmverdampfer vorgeschaltet. Hier wird die Packungskolonne aus den Brüden des Fallfilmverdampfers gespeist. In beiden Fällen soll durch den Fallfilmverdampfer sichergestellt werden, dass möglichst wenig Wertprodukt (meta-Diaminotoluol) gemeinsam mit hochsiedenden Rückständen verloren geht. In beiden Fällen wird das Sumpfprodukt des Fallfilmverdampfers (im Wesentlichen bestehend aus hochsiedenden Rückständen) mit einem im oberen Teil der Packungskolonne entnommenen kondensierten ortho-Diaminotoluolstrom vermischt.

EP 0 696 574 B1 offenbart in den Beispielen 9 und 10 ein Verfahren, in dem das Produkt der Nitrobenzolhydrierung einschließlich des Reaktionswassers in einer Destillationskolonne destilliert und das Sumpfprodukt der Destillationskolonne mit der nach Phasentrennung des Kondensats des Kopfprodukts derselben Destillationskolonne erhaltenen Anilin-reichen Phase verdünnt wird.

In EP 1 005 888 B1 wird eine Spülvorrichtung zur Entfernung von Rückständen aus dem Sumpfablauf einer Verdampfungseinrichtung und ihre Verwendung für die destillative Aufarbeitung salzhaltiger Lösungen beschrieben. Nachteilig ist, dass wieder ein gewisser Aufwand mit dem gebrauchten Spülmittel getrieben werden muss, was bis dahin geht, dass es teuer verbrannt werden muss. Darüber hinaus eignet sich eine solche Spülvorrichtung nicht, wenn am Sumpf der Kolonne hohe Temperaturen herrschen, so dass hochsiedende Lösungsmittel verwendet werden müssten oder der Sumpf abgekühlt werden müsste, um den Spülvorgang ohne Verdampfung des Spülmittels durchzuführen. Bevorzugt wird im beschriebenen Verfahren Wasser eingesetzt, was jedoch für organische Rückstände nur bedingt als Spülmittel geeignet ist. Zudem lässt es sich in der Praxis nicht immer vermeiden, dass Spülmittel in die Kolonne gelangt und die Qualität des Kopfprodukts und/oder die weitere Aufarbeitung des Kopfprodukts beeinträchtigt.

Aufgabe der vorliegenden Erfindung war es daher ein Verfahren bereit zu stellen, welches ein reibungsloses Betreiben der Aufarbeitungskolonnen gewährleistet und gleichzeitig die Anilinverluste minimiert.

Erfindungsgemäß gelöst wird diese Aufgabe durch ein Verfahren zur Behandlung eines ein aromatisches Amin umfassenden Stoffgemisches, wobei das aromatische Amin Anilin oder 2,4-Diaminotoluol ist,
wobei das Stoffgemisch ein erstes Stoffgemisch ist und ein aromatisches Amin (nämlich Anilin oder 2,4-Diaminotoluol) und Verbindungen mit einem höheren Siedepunkt als das aromatische Amin umfasst, wobei das erste Stoffgemisch vorzugsweise entwässert ist, wobei die Entwässerung durch Phasentrennung und/oder Destillation erfolgen kann,
umfassend den Schritt:
I) Destillatives Auftrennen des ersten Stoffgemisches in einer ersten Destillationseinheit unter zumindest teilweiser Abtrennung des aromatischen Amins, wobei weiterhin ein erstes Sumpfprodukt erhalten wird und dieses erste Sumpfprodukt aus der ersten Destillationseinheit ausgetragen wird;
wobei das Sumpfprodukt aus der ersten Destillationseinheit nach dem Austragen mit einem kondensierten Kopfprodukt aus einer von der ersten Destillationseinheit verschiedenen Destillationseinheit und/oder mit einer Methanol umfassenden Zusammensetzung verdünnt wird.

Vorteilhaft an dieser erfindungsgemäßen Verfahrensweise ist, dass aufgrund des Verdünnungseffekts die Konzentration des gewünschten aromatischen Amins im Rückstand (Sumpfprodukt aus der ersten Destillationseinheit) gesenkt werden kann, da das Amin als Lösungsmittel nicht mehr benötigt wird. Die hochsiedenden Rückstände können also im Sumpf der Kolonne weiter aufkonzentriert und es kann so die Amin-Ausbeute erhöht werden. Auch kann auf eine energieaufwändige Beheizung eines Rückstandsbehälters und die üblichen Rohrbegleitheizungen verzichtet werden.

Im Sinne der vorliegenden Erfindung umfasst eine *Destillationseinheit* eine Destillationskolonne und die dazu gehörigen Peripheriegeräte wie beispielsweise Verdampfer, wobei ein Verdampfer auch in der Destillationskolonne integriert sein kann. Die erste Destillationseinheit kann beispielsweise eine Reindestillationskolonne, insbesondere eine Anilin-Reindestillationskolonne sein. Weiterhin kann die erste Destillationseinheit auch im Verbund mit anderen Destillationseinheiten eingesetzt werden, so dass sie am Ende einer mehrstufigen Destillation als Schwersiederkolonne ausgeführt ist. Obwohl die Anforderungen an eine solche Schwersiederkolonne aufgrund der physikalischen Randbedingungen (Vakuum, Temperatur, Viskosität) höher sind, ergeben sich hieraus Vorteile, da nicht mehr der gesamte Massenstrom an aromatischem Amin in der Kolonne destilliert wird, und die technisch aufwändigeren Apparate deshalb kleiner gestaltet werden können.

Zweckmäßigerweise wird das Sumpfprodukt in einen Behälter ausgetragen und dort dann verdünnt. Es ist aber auch denkbar, dass das Verdünnen in einer außerhalb der ersten Destillationseinheit gelegenen Rohrleitung stattfindet.

Das kondensierte Kopfprodukt aus einer Destillationseinheit kann aus der ersten Destillationseinheit und/oder aus einer von der ersten Destillationseinheit verschiedenen Destillationseinheit gewonnen werden.

Der Rückstand (Sumpfprodukt) wird einerseits durch das verbliebene Amin und andererseits durch ausreichend zusätzlich eingebrachte Verdünnungsmittel (Methanol umfassende Zusammensetzung und/oder als Kopfprodukte von Destillationseinheiten, die von der ersten Destillationseinheit verschiedenen sind, anfallende Leichtsieder) flüssig gehalten. Dies verhindert außerdem Verstopfungen in der Rohrleitung und in den Lanzen zu einer Verbrennung. Die Bilanz stimmt, da die Methanol umfassende Zusammensetzung und/oder die Leichtsieder, welche insbesondere aus einem Polyisocyanat-Verbund stammen (zum Beispiel einem MDA-Betrieb) und ohnehin entsorgt werden müssen, nun die Aminrückgewinnung ermöglichen. Im Rahmen der vorliegenden Erfindung bezeichnet der Begriff "Leichtsieder" vorzugsweise Stoffe oder Stoffgemische mit einem Siedepunkt bei 1013 mbar von ≥ 30 °C bis ≤ 220 °C, bevorzugt von ≥ 50 °C bis ≤ 197 °C, besonders bevorzugt von ≥ 50 °C bis ≤ 185 °C, ganz besonders bevorzugt von ≥ 60 °C bis < 100 °C.

Andere denkbare Quellen für diese Verdünnungs-Flüssigkeit sind aus der Amin-Destillation abgetrennte Leichtsieder, aliphatenreiche Abfallströme aus dem Nitrobenzol-Prozess (vgl. DE 10 2009 005324 A1) sowie abgetrennte Leichtsieder aus einem TDA-Prozess (zum Beispiel 1,3-Diamino-4-methylcyclohexan).

Wenn Leichtsieder und/oder Methanolwasser (beispielsweise aus der MDA-Herstellung) im Rückstandsbehälter zur Verdünnung des Rückstandes (Sumpfprodukt aus der ersten Destillationseinheit) beigemischt werden, ergeben sich folgende Vorteile:
i) Durch den Verdünnungseffekt ist es möglich, die Amin-Konzentration des Rückstands zu senken
ii) Die hochsiedenden Rückstände können also im Sumpf der Kolonne weiter auf konzentriert und so die Amin-Ausbeute erhöht werden.
iii) Der Rückstand wird durch ausreichend Leichtsieder und/oder Methanol flüssig gehalten, wodurch sich die Lanzen einer thermischen Abluftreinigung (TAR) nicht zusetzen.
iv) Es gibt außerdem eine Energieeinsparung, weil der Rückstand bei niedrigerer Temperatur gelagert werden kann und Rohrbegleitheizungen entfallen können.
v) Die Entsorgungsbilanz stimmt, da solche Flüssigkeiten eingesetzt werden, die ohnehin entsorgt werden müssen.
vi) Da die Verdünnung erst einem Rückstandsbehälter erfolgt, kommt es zu keiner Beeinträchtigung der Destillation.

Ausführungsformen der vorliegenden Erfindung werden nachfolgend beschrieben. Sie können beliebig kombiniert werden, sofern sich aus dem Kontext nicht eindeutig das Gegenteil ergibt.

Auch wenn im Folgenden exemplarisch die Herstellung von aromatischen Aminen durch Gasphasenhydrierung erwähnt wird, lässt das erfindungsgemäße Verfahren natürlich auf jedes anders hergestellte, aromatische Amine umfassende Stoffgemisch anwenden, also insbesondere auch auf aromatische Amine umfassende Stoffgemische, die in Flüssigphasenprozessen hergestellt wurden oder solche, in denen die Einsatzstoffe an Katalysator-Wirbelschichten umgesetzt werden.

In einer Ausführungsform des erfindungsgemäßen Verfahrens umfasst dieses weiterhin die Schritte Ia) und Ib), wobei Ia) und Ib) vor I) durchgeführt werden:
Ia) Bereitstellen eines zweiten Stoffgemischs, wobei das zweite Stoffgemisch das aromatische Amin, Verbindungen mit einem niedrigeren Siedepunkt als das aromatische Amin und Verbindungen mit einem höheren Siedepunkt als das aromatische Amin umfasst und der Gehalt an aromatischem Amin vom Gehalt an aromatischem Amin im ersten Stoffgemisch verschieden ist;
Ib) Destillatives Auftrennen des zweiten Stoffgemischs in einer vorgelagerten Destillationseinheit unter Abtrennung von Verbindungen mit einem niedrigeren Siedpunkt als das aromatische Amin als Kopfprodukt, wobei weiterhin das aromatische Amin als Seitenstrom und das erste Stoffgemisch als Sumpfprodukt erhalten wird. Das im erfindungsgemäßen Verfahren zu behandelnde Stoffgemisch wird als Sumpfprodukt erhalten, welches selbstverständlich auch noch das aromatische Amin enthält. Die erste Destillationseinheit des erfindungsgemäßen Verfahrens ist in diesem Fall vorzugsweise eine Rückstandskolonne.

In einer weiteren Ausführungsform des erfindungsgemäßen Verfahrens umfasst dieses weiterhin die Schritte IIa), IIb) und III), wobei IIa) und IIb) vor III) sowie III) vor I) durchgeführt werden:
IIa) Bereitstellen eines zweiten Stoffgemischs, wobei das zweite Stoffgemisch das aromatische Amin, Verbindungen mit einem niedrigeren Siedepunkt als das aromatische Amin und Verbindungen mit einem höheren Siedepunkt als das aromatische Amin umfasst und der Gehalt an aromatischem Amin vom Gehalt an aromatischem Amin im ersten Stoffgemisch verschieden ist;
IIb) Destillatives Auftrennen des zweiten Stoffgemischs in einer zweiten Destillationseinheit unter Abtrennung von Verbindungen mit einem niedrigeren Siedpunkt als das aromatische Amin als Kopfprodukt, wobei weiterhin ein das aromatische Amin umfassendes Sumpfprodukt erhalten wird;
III) Destillatives Auftrennen des Sumpfprodukts aus der zweiten Destillationseinheit in einer dritten Destillationseinheit unter zumindest teilweiser Abtrennung des aromatischen Amins als Kopfprodukt, wobei weiterhin das erste Stoffgemisch als Sumpfprodukt erhalten wird.

Somit ergibt sich in dieser Ausführungsform die folgende Reihenfolge der Schritte: IIa), IIb), III), I). Es wird auch deutlich, dass die Bezeichnungen "erste Destillationseinheit", "zweite Destillationseinheit" und "dritte Destillationseinheit" keine Reihenfolge der Destillationseinheiten im erfindungsgemäßen Verfahren darstellen. Vielmehr kann die zweite Destillationseinheit eine Leichtsiederkolonne sein, gefolgt von der dritten Destillationseinheit als Amin-Reinkolonne (insbesondere Anilinreinkolonne), woran sich die erste Destillationseinheit als Schwersiederkolonne anschließt.

In einer bevorzugten Ausführungsform des erfindungsgemäßen Verfahrens beträgt der Gehalt des aromatischen Amins im Sumpfprodukt aus der ersten Destillationseinheit ≥ 5 Gewichts-% bis ≤ 70 Gewichts-%, besonders bevorzugt ≥ 10 Gewichts-% bis ≤ 45 Gewichts-%, jeweils bezogen auf das Gesamtgewicht des Sumpfprodukts. Hierunter ist der Gehalt des aromatischen Amins im Sumpfprodukt zu verstehen, nachdem das Sumpfprodukt ausgetragen wurde und bevor das Sumpfprodukt verdünnt wird.

Erfindungsgemäß wird das Sumpfprodukt aus der ersten Destillationseinheit nach dem Austragen mit dem Kopfprodukt aus einer von der ersten Destillationseinheit verschiedenen Destillationseinheit und/oder mit einer Methanol umfassenden Zusammensetzung verdünnt. In einer bevorzugten Ausführungsform des erfindungsgemäßen Verfahrens wird das Sumpfprodukt aus der ersten Destillationseinheit nach dem Austragen mit dem Kopfprodukt aus einer von der ersten Destillationseinheit verschiedenen Destillationseinheit und/oder mit der Methanol umfassenden Zusammensetzung so verdünnt, dass die erhaltene Mischung eine dynamische Viskosität bei 20 °C von ≥ 0,3 mPas bis ≤ 1000 mPas, bevorzugt von ≥ 0,5 mPas bis ≤ 100 mPas, besonders bevorzugt von ≥ 1 mPas bis ≤ 50 mPas, ganz besonders bevorzugt von ≥ 1 mPas bis ≤ 10 mPas aufweist. Die Viskosität wird hierbei gemessen mittels Kugelfallviskosimeter gemäß DIN 53015/ISO 12058.

Es ist bevorzugt, dass das kondensierte Kopfprodukt aus der von der ersten Destillationseinheit verschiedenen Destillationseinheit bei der Temperatur, bei welcher das Sumpfprodukt aus der ersten Destillationseinheit nach dem Austragen verdünnt wird, einphasig ist, d. h. Wasser nur bis zur Sättigungsgrenze enthält, sodass eine spontane Entmischung in eine wässrige und eine organische Phase vermieden wird. Auf diese Weise bleibt bevorzugt auch das verdünnte Sumpfprodukt der ersten Destillationseinheit einphasig. In einer weiteren Ausführungsform des erfindungsgemäßen Verfahrens enthält das kondensierte Kopfprodukt aus der von der ersten Destillationseinheit verschiedenen Destillationseinheit daher bevorzugt ≥ 80 Gewichts-% bis ≤ 100 Gewichts-% Leichtsieder und ≥ 0 Gewichts-% bis ≤ 20 Gewichts-% Wasser, bezogen auf das Gesamtgewicht des Kopfprodukts. Besonders bevorzugt besteht das kondensierte Kopfprodukt aus den zuvor genannten Anteilen an Leichtsiedern und Wasser.

In einer weiteren Ausführungsform des erfindungsgemäßen Verfahrens ist die Methanol umfassende Zusammensetzung eine Methanol und Wasser umfassende Zusammensetzung, welche aus einem Verfahren zur Herstellung von Methylendiphenyldiamin (MDA) gewonnen wurde.

Das sogenannte Methanolwasser fällt bei der Produktion von MDA an. Üblicherweise stehen die Anlagen zur Herstellung von Anilin und MDA sehr nahe beieinander, da sie Teil der Herstellungskette von MDI sind. Der Transportweg von Methanolwasser von MDA zu Anilin ist daher sehr kurz und kann per Rohrleitung vollzogen werden. Methanolwasser fällt in der Reaktion des MDA-Prozesses an.

In EP 1 616 890 A1 ist die saure Kondensation von aromatischen Aminen und Formaldehyd zu MDA beschrieben. In Abwesenheit eines sauren Katalysators kondensiert zunächst Formaldehyd, - technisch verfügbares Formaldehyd enthält Methanol zur Stabilisierung - mit Anilin zu sogenanntem Aminal und Wasser. Die Umlagerung zum MDA erfolgt säurekatalysiert in einem ersten Schritt zu para- bzw. ortho-Aminobenzylanilin. Die Aminobenzylaniline lagern in einem zweiten Schritt zum MDA um. Nach der Umsetzung zum Aminal wird zunächst das Wasser zumindest teilweise und das Methanol zur Gänze als sogenanntes Methanolwasser aus dem Aminal entfernt und anschließend das Aminal mit saurem Katalysator versetzt und das so erhaltenen saure Reaktionsgemisch bei Temperaturen von 20 °C bis 100 °C weiter umgesetzt. Der Wassergehalt liegt dabei zwischen 0 und 20 Gew.-%.

In einer weiteren Ausführungsform des erfindungsgemäßen Verfahrens enthält die Methanol umfassende Zusammensetzung ≥ 20 Gewichts-% bis ≤ 95 Gewichts-% Methanol, bezogen auf das Gesamtgewicht der Methanol umfassenden Zusammensetzung. Vorzugsweise beträgt der Anteil ≥ 50 Gewichts-% bis ≤ 80 Gewichts-% Methanol, bezogen auf das Gesamtgewicht der Methanol umfassenden Zusammensetzung. Es ist weiterhin bevorzugt, dass der Rest der Zusammensetzung Wasser sowie technisch unvermeidliche Verunreinigungen, insbesondere Salze, etc. enthält.

In einer weiteren Ausführungsform des erfindungsgemäßen Verfahrens ist der Mengenstrom der Methanol umfassenden Zusammensetzung so gewählt, dass der rechnerische Mengenstrom an reinem Methanol ≥ 30 Gewichts-%, bezogen auf den Mengenstrom an ausgetragenem Sumpfprodukt aus der ersten Destillationseinheit beträgt. Vorzugsweise beträgt dieser Mengenstrom ≥ 40 Gewichts-%.

In einer weiteren Ausführungsform des erfindungsgemäßen Verfahrens kann es bevorzugt sein, zur Verdünnung des Sumpfproduktes aus der ersten Destillationseinheit nur eine Methanol umfassende Zusammensetzung zu verwenden, d. h. auf die Zufuhr des kondensierten Kopfproduktes einer anderen Destillationseinheit ganz zu verzichten. Dieser Fall kann insbesondere dann eintreten, wenn aus einer benachbarten MDA-Anlage eine ausreichende Menge einer Methanol umfassenden Zusammensetzung bereitgestellt werden kann. Diese Ausführungsform kann auch dann bevorzugt sein, wenn die Zugabe des kondensierten Kopfprodukts einer anderen Destillationseinheit zu einer spontanen Phasentrennung des verdünnten Sumpfproduktes aus der ersten Destillationseinheit führen würde.

In einer weiteren Ausführungsform des erfindungsgemäßen Verfahrens wird das Sumpfprodukt aus der dritten Destillationseinheit nach dem Verdünnen verbrannt.

Die vorliegende Erfindung wird anhand der nachfolgenden Figuren und Beispiele näher erläutert, ohne jedoch darauf beschränkt zu sein.
FIG. 1 zeigt schematisch eine Anlage und ein Verfahren zur Aufarbeitung des Rohprodukts aus einer Anilinherstellung
FIG. 2 zeigt schematisch eine weitere Anlage und ein weiteres Verfahren zur Aufarbeitung des Rohprodukts aus einer Anilinherstellung

Im Verfahrensschema gemäß FIG. 1 gelangt Rohanilin entweder direkt aus einer Produktionsanlage/Zwischenlagerung (Stoffstrom 200) und/oder aus einem Phasentrennungsbehälter 100 (Stoffstrom 210) in eine erste Destillationseinheit 110, welche als Anilin-Reindestillationskolonne ausgeführt ist. Bei Bedarf kann auch wässrige Base, bevorzugt Natronlauge, (Stoffstrom 220) hinzugefügt werden. Die Reindestillations-Kolonne 110 wird bei vermindertem Druck betrieben; die notwendige Verdampfungsenergie kann über Umlaufverdampfer zugeführt werden, die mit Dampf beheizt werden. Der benötigte verminderte Druck wird mit einer Flüssigkeitsringpumpe erzeugt. Als Betriebsflüssigkeit für die Vakuumpumpen dient Anilin-Wasser.

Das Reinanilin wird zum Teil mittels Reinanilin-Pumpen als Rücklauf auf die Kolonne gegeben. Der übrige Teil wird nach Abkühlung als Produkt abgeführt (Stoffstrom 230).

Niedrigsiedende Destillationsprodukte werden der Kolonne 110 als Kopfstrom 240 entnommen und teilweise kondensiert. Über einen Phasentrenner 120 wird der organische, anilinreiche Teil des Kondensats auf den Kolonnenkopf zurückgegeben. Der wässrige Teil des Kondensats wird als Stoffstrom 250 zurück in den Phasentrennungsbehälter 100 gegeben.

Im Kolonnensumpf bleiben die aufkonzentrierten Schwersieder zurück. Sie werden als Stoffstrom 260 ausgeschleust und in der Rückstandsvorlage 140 gesammelt. Dort werden sie mit MethanolWasser aus einem MDA-Betrieb (Stoffstrom 270) verdünnt und, gegebenenfalls zusammen mit Leichtsiedern, einer thermischen Verwertung (Stoffstrom 280) zugeführt.

Die in dem Phasentrennungsbehälter 100 separierte wässrige Phase enthält gelöstes Anilin und wird als Stoffstrom 290 auf eine als Stripp- Kolonne 150 ausgeführte Destillationseinheit gegeben. Das Kopfprodukt aus dieser Destillation umfasst ein Azeotrop aus Wasser und Anilin und kann dann als Stoffstrom 300 in den Phasentrennungsbehälter 100 zurückgeführt werden. Am Sumpf der Kolonne wird weitgehend von Anilin befreites Wasser als Abwasser (Stoffstrom 310) dem Prozess entnommen.

Im Verfahrensschema gemäß FIG. 2 gelangt Rohanilin entweder direkt aus einer Produktionsanlage/Zwischenlagerung (Stoffstrom 1200) und/oder aus einem Phasentrennungsbehälter 1100 (Stoffstrom 1210) in eine in der Terminologie der Erfindung als zweite Destillationseinheit 1110 bezeichnete Kolonne, welche als Destillationskolonne oder Entwässerungskolonne zur Abtrennung von Niedrigsiedern dient. Die Entwässerungs-Kolonne 1110 wird bei Normaldruck betrieben. Die notwendige Verdampfungsenergie kann über einen dampfbeheizten Umlaufverdampfer zugeführt werden. Auf den mittleren Kolonnenböden reichert sich ein Wasser/Anilin-Azeotrop, am Kolonnenkopf ein Gemisch aus Wasser, Anilin und Leichtsiedern an. Das Kopfprodukt wird nach Kondensation in Kondensatoren auf den Kolonnenkopf zurückgeführt, nicht kondensierte Anteile werden als Abgas entsorgt.

Das Wasser/Anilin-Azeotrop wird der Kolonne 1110 als Seitenstrom 1220 entnommen und zur Phasentrennung in den Trennbehälter 1100 zurückgeführt.

Das Rohanilin am Kolonnensumpf der Kolonne 1110 ist weitgehend von Leichtsiedern und Wasser befreit und wird als Stoffstrom 1230 einer weiteren Kolonne, in der Terminologie der Erfindung als dritte Destillationseinheit 1120 bezeichnet, zugeführt und dort weiter fraktioniert. Am Kopf dieser Reindestillations-Kolonne 1120 reichert sich Anilin an, im Sumpf die Schwersieder. Die Reinanilin-Dämpfe aus dem Kolonnen-Kopf werden auskondensiert.

Die Reindestillations-Kolonne 1120 wird bei vermindertem Druck betrieben; die notwendige Verdampfungsenergie kann über Umlaufverdampfer zugeführt werden, die mit Dampf beheizt werden. Das gesammelte Reinanilin wird zum Teil mittels Reinanilin-Pumpen als Rücklauf auf den Kolonnenkopf gegeben.

Der mit Schwersiedern angereicherte Sumpf der Reindestillations-Kolonne 1120 wird zur weiteren Aufkonzentrierung als Stoffstrom 1240 in die als Rückstands-Kolonne 1130 ausgeführte, in der Terminologie der Erfindung als erste Destillationseinheit bezeichnete Kolonne, gefördert (Rückstandsdestillation). Die Rückstands-Kolonne 1130 arbeitet ebenfalls bei vermindertem Druck. Die notwendige Verdampfungsenergie kann über einen dampfbeheizten Fallfilmverdampfer zugeführt werden. Am Kolonnenkopf geht Anilin-Dampf über, der in einem Kondensator auskondensiert. Ein Teil des Kondensats wird mittels Destillat-Pumpen als Rücklauf auf den Kolonnenkopf gegeben. Das übrige Kondensat kann je nach Qualität entweder über einen Wärmeaustauscher abgegeben oder dem Zulauf der Reinanilin-Kolonne 1120 als Stoffstrom 1250 beigemischt werden.

Im Kolonnensumpfbleiben die aufkonzentrierten Schwersieder zurück. Sie werden als Stoffstrom 1260 ausgeschleust und in der Rückstandsvorlage 1140 gesammelt. Dort werden sie mit MethanolWasser aus einem MDA-Betrieb (Stoffstrom 1270) verdünnt und, gegebenenfalls zusammen mit weiteren Leichtsiedern, einer thermischen Verwertung (Stoffstrom 1280) zugeführt.

Die in dem Phasentrennungsbehälter 1100 separierte wässrige Phase enthält gelöstes Anilin und wird als Stoffstrom 1290 auf eine als Stripp-Kolonne 1150 ausgeführte vierte Destillationseinheit gegeben. Das Kopfprodukt aus dieser Destillation umfasst ein Azeotrop aus Wasser und Anilin und kann dann als Stoffstrom 1300 in den Phasentrennungsbehälter 1100 zurückgeführt werden. Am Sumpf der Kolonne wird weitgehend von Anilin befreites Wasser als Abwasser (Stoffstrom 1310) dem Prozess entnommen.

### Beispiele:

Beispiel 1 (Vergleichsbeispiel): normaler Anilinverlust (328 Tonnen pro Jahr).

Eine an einen Anilin-Prozess angeschlossene Aufarbeitung mit einer Leichtsiederkolonne, einer Reinanilin-Kolonne und einer Rückstandskolonne wurde so betrieben (FIG. 2), dass sich im Sumpf der Rückstandskolonne (1130, erste Destillationseinheit) ein Restanilin-Gehalt von ca. 50 % einstellte. Der aus dem Sumpf ausgeschleuste Rückstand wurde bei 35 °C gelagert und dann in einer Verbrennung entsorgt. Die relevanten Kenndaten sind in Tabelle 1 wiedergegeben.

**Tabelle 1:**

| **Mengenstrom Anilin zur Rückstandskolonne (Strom 1240)** | **Anteil Schwersieder** | **Ausschleusung Sumpf (1260)** | **Verlust Anilin** |
|---|---|---|---|
| 800 kg/h | 5 % | 80 kg/h | 40 kg/h |

Beispiel 2 (Vergleichsbeispiel): geringerer Anilinverlust (theoretisch 56 Tonnen pro Jahr) mit Verstopfung der Rohrleitung und der Lanzen in der TAR.

Eine an einen Anilin-Prozess angeschlossene Aufarbeitung mit einer Leichtsiederkolonne, einer Reinanilin-Kolonne und einer Rückstandskolonne wird so betrieben (FIG. 2), dass sich im Sumpf der Rückstandskolonne ein Restanilin-Gehalt von 30 % einstellte. Der aus dem Sumpf ausgeschleuste Rückstand wurde bei 120 °C gelagert und dann in einer Verbrennung entsorgt. Hierbei kam es trotz Begleitheizung der Rohrleitung zur Verbrennung zu Ablagerungen in dieser Rohrleitung und zu Verstopfungen in der Lanze der Verbrennungsanlage. Der Prozess musste häufig unterbrochen werden, um einen auf der Saugseite der Rückstandspumpe angeordneten Schmutzfänger sowie die Lanze in der Verbrennungsanlage zu reinigen. Während dieser Reinigungsarbeiten wurde die Beheizung des Rückstandsbehälters abgestellt. Beim Erkalten des Rückstandsgemisches kam es zu einem Viskositätsanstieg, der dazu führte, dass das Gemisch nicht mehr pumpfähig war. Die relevanten Kenndaten sind in Tabelle 2 wiedergegeben.

**Tabelle 2:**

| **Mengenstrom Anilin zur Rückstandskolonne (Strom 1240)** | **Anteil Schwersieder** | **Ausschleusung Sumpf (1260)** | **Verlust Anilin** |
|---|---|---|---|
| 800 kg/h | 5 % | 57 kg/h | 17 kg/h |

Beispiel 3 (erfindungsgemäßes Beispiel): Verdünnen des Rückstands im Behälter mit Methanol.

Eine an einen Anilin-Prozess angeschlossene Aufarbeitung mit einer Leichtsiederkolonne, einer Reinanilin-Kolonne und einer Rückstandskolonne wurde so betrieben (FIG. 2), dass sich im Sumpf der Rückstandskolonne ein Restanilin-Gehalt von ca. 30 Gewichts-% einstellte. Der aus dem Sumpf ausgeschleuste Rückstand wurde bei 35 °C gelagert und mit der ca. 0,6-fachen Menge (Massenstrom), bezogen auf den ausgeschleusten Rückstand, an Methanolwasser (72 Gewichts-% Methanol) aus einem benachbarten Methylendiphenyldiamin- (MDA-)Betrieb verdünnt. Bei der Entsorgung des so behandelten Rückstands gab es keine Probleme mit Verstopfungen oder Ablagerungen. Die relevanten Kenndaten sind in Tabelle 3 wiedergegeben.

**Tabelle 3:**

| **Mengenstrom Anilin zur Rückstandskolonne (Strom 1240)** | **Anteil Schwersieder** | **Ausschleusung Sumpf (1260)** | **Zugabe Methanolwasser (1270)** | **Verlust Anilin** |
|---|---|---|---|---|
| 800 kg/h | 5 % | 57 kg/h | 35 kg/h | 17 kg/h |

## Patentansprüche

1. Verfahren zur Behandlung eines ein aromatisches Amin umfassenden Stoffgemisches, wobei das aromatische Amin Anilin oder 2,4-Diaminotoluol ist,
wobei das Stoffgemisch ein erstes Stoffgemisch ist und ein aromatisches Amin und Verbindungen mit einem höheren Siedepunkt als das aromatische Amin umfasst,
umfassend den Schritt:
I) Destillatives Auftrennen des ersten Stoffgemisches in einer ersten Destillationseinheit (110, 1130) unter zumindest teilweiser Abtrennung des aromatischen Amins, wobei weiterhin ein erstes Sumpfprodukt erhalten wird und dieses erste Sumpfprodukt aus der ersten Destillationseinheit (110, 1130) ausgetragen wird;
**dadurch gekennzeichnet, dass**
das Sumpfprodukt aus der ersten Destillationseinheit (110, 1130) nach dem Austragen mit einem kondensierten Kopfprodukt aus einer von der ersten Destillationseinheit verschiedenen Destillationseinheit und/oder mit einer Methanol umfassenden Zusammensetzung verdünnt wird.

2. Verfahren gemäß Anspruch 1,
weiterhin umfassend die Schritte Ia) und Ib), wobei Ia) und Ib) vor I) durchgeführt werden:
Ia) Bereitstellen eines zweiten Stoffgemischs, wobei das zweite Stoffgemisch das aromatische Amin, Verbindungen mit einem niedrigeren Siedepunkt als das aromatische Amin und Verbindungen mit einem höheren Siedepunkt als das aromatische Amin umfasst und der Gehalt an aromatischem Amin vom Gehalt an aromatischem Amin im ersten Stoffgemisch verschieden ist;
Ib) Destillatives Auftrennen des zweiten Stoffgemischs in einer vorgelagerten Destillationseinheit unter Abtrennung von Verbindungen mit einem niedrigeren Siedpunkt als das aromatische Amin als Kopfprodukt, wobei weiterhin das aromatische Amin als Seitenstrom und das erste Stoffgemisch als Sumpfprodukt erhalten wird.

3. Verfahren gemäß Anspruch 1,
weiterhin umfassend die Schritte IIa), IIb) und III), wobei IIa) und IIb) vor III) sowie III) vor I) durchgeführt werden:
IIa) Bereitstellen eines zweiten Stoffgemischs, wobei das zweite Stoffgemisch das aromatische Amin, Verbindungen mit einem niedrigeren Siedepunkt als das aromatische Amin und Verbindungen mit einem höheren Siedepunkt als das aromatische Amin umfasst, und der Gehalt an aromatischem Amin vom Gehalt an aromatischem Amin im ersten Stoffgemisch verschieden ist;
IIb) Destillatives Auftrennen des zweiten Stoffgemischs in einer zweiten Destillationseinheit (1110) unter Abtrennung von Verbindungen mit einem niedrigeren Siedpunkt als das aromatische Amin als Kopfprodukt, wobei weiterhin ein das aromatische Amin umfassendes Sumpfprodukt erhalten wird;
III) Destillatives Auftrennen des Sumpfprodukts aus der zweiten Destillationseinheit in einer dritten Destillationseinheit (1120) unter zumindest teilweiser Abtrennung des aromatischen Amins als Kopfprodukt, wobei weiterhin das erste Stoffgemisch als Sumpfprodukt erhalten wird.

4. Verfahren gemäß einem der Ansprüche 1 bis 3, wobei der Gehalt des aromatischen Amins im Sumpfprodukt aus der ersten Destillationseinheit (110, 1130) ≥ 5 Gewichts-% bis ≤ 70 Gewichts-% beträgt.

5. Verfahren gemäß einem der Ansprüche 1 bis 4, wobei das Sumpfprodukt aus der ersten Destillationseinheit (110, 1130) nach dem Austragen mit dem Kopfprodukt aus einer von der ersten Destillationseinheit verschiedenen Destillationseinheit und/oder mit der Methanol umfassenden Zusammensetzung so verdünnt wird, dass die erhaltene Mischung eine Viskosität bei 20 °C von ≥ 0,3 mPas bis ≤ 1000 mPas aufweist.

6. Verfahren gemäß einem der Ansprüche 1 bis 5, wobei die Methanol umfassende Zusammensetzung eine Methanol und Wasser umfassende Zusammensetzung ist, welche aus einem Verfahren zur Herstellung von Methylendiphenyldiamin gewonnen wurde.

7. Verfahren gemäß einem der Ansprüche 1 bis 6, wobei die Methanol umfassende Zusammensetzung ≥ 20 Gewichts-% bis ≤ 95 Gewichts-% Methanol enthält.

8. Verfahren gemäß einem der Ansprüche 1 bis 7, wobei der Mengenstrom der Methanol umfassenden Zusammensetzung so gewählt ist, dass der rechnerische Mengenstrom an reinem Methanol ≥ 30 Gewichts, bezogen auf den Mengenstrom an ausgetragenem Sumpfprodukt aus der ersten Destillationseinheit (110, 1130) beträgt.

9. Verfahren gemäß einem der Ansprüche 1 bis 8, wobei das aus einer von der ersten Destillationseinheit verschiedenen Destillationseinheit stammende kondensierte Kopfprodukt ≥ 80 Gewichts-% bis ≤ 100 Gewichts-% Leichtsieder und ≥ 0 Gewichts-% bis ≤ 20 Gewichts-% Wasser enthält.

10. Verfahren gemäß einem der Ansprüche 1 bis 8, wobei das Sumpfprodukt aus der ersten Destillationseinheit (110, 1130) nach dem Austragen nur mit einer Methanol umfassenden Zusammensetzung verdünnt wird.

11. Verfahren gemäß einem der Ansprüche 1 bis 10, wobei das Sumpfprodukt aus der ersten Destillationseinheit (110, 1130) nach dem Verdünnen verbrannt wird.

## Claims

1. Process for treating a substance mixture comprising an aromatic amine, wherein the aromatic amine is aniline or 2,4-diaminotoluene,
wherein the substance mixture is a first substance mixture and comprises an aromatic amine and compounds having a higher boiling point than the aromatic amine, comprising the step of:
I) distillatively separating the first substance mixture in a first distillation unit (110, 1130) to separate off at least some of the aromatic amine and to additionally obtain a first bottom product, this first bottom product being discharged from the first distillation unit (110, 1130);
**characterized in that**
once discharged the bottom product from the first distillation unit (110, 1130) is diluted with a condensed top product from a distillation unit distinct from the first distillation unit and/or with a composition comprising methanol.

2. Process according to Claim 1,
further comprising the steps Ia) and Ib), wherein Ia) and Ib) are carried out prior to I):
Ia) providing a second substance mixture, wherein the second substance mixture comprises the aromatic amine, compounds having a lower boiling point than the aromatic amine and compounds having a higher boiling point than the aromatic amine and the aromatic amine content is different from the aromatic amine content in the first substance mixture;
Ib) distillatively separating the second substance mixture in an upstream distillation unit to separate off compounds having a lower boiling point than the aromatic amine as top product and to additionally obtain the aromatic amine as sidestream and the first substance mixture as bottom product.

3. Process according to Claim 1,
further comprising the steps IIa), IIb) and III), wherein IIa) and IIb) are carried out prior to III) and III) is carried out prior to I):
IIa) providing a second substance mixture, wherein the second substance mixture comprises the aromatic amine, compounds having a lower boiling point than the aromatic amine and compounds having a higher boiling point than the aromatic amine and the aromatic amine content is different from the aromatic amine content in the first substance mixture;
IIb) distillatively separating the second substance mixture in a second distillation unit (1110) to separate off compounds having a lower boiling point than the aromatic amine as top product and to additionally obtain a bottom product comprising the aromatic amine;
III) distillatively separating the bottom product from the second distillation unit in a third distillation unit (1120) to separate off at least some of the aromatic amine as top product and to additionally obtain the first substance mixture as bottom product.

4. Process according to any one of Claims 1 to 3, wherein the content of the aromatic amine in the bottom product from the first distillation unit (110, 1130) is ≥ 5% by weight to ≤ 70% by weight.

5. Process according to any one of Claims 1 to 4, wherein once discharged the bottom product from the first distillation unit (110, 1130) is diluted with the top product from a distillation unit distinct from the first distillation unit and/or with the composition comprising methanol such that the mixture obtained has a viscosity at 20°C of from ≥ 0.3 mPas to ≤ 1000 mPas.

6. Process according to any one of Claims 1 to 5, wherein the composition comprising methanol is a composition comprising methanol and water which has been obtained from a process for producing methylenediphenyldiamine.

7. Process according to any one of Claims 1 to 6, wherein the composition comprising methanol comprises ≥ 20% by weight to ≤ 95% by weight of methanol.

8. Process according to any one of Claims 1 to 7, wherein the flow rate of the composition comprising methanol is chosen such that the theoretical flow rate of pure methanol is ≥ 30% by weight based on the flow rate of discharged bottom product from the first distillation unit (110, 1130).

9. Process according to any one of Claims 1 to 8, wherein the condensed top product originating from a distillation unit distinct from the first distillation unit comprises ≥ 80% by weight to ≤ 100% by weight of low-boilers and ≥ 0% by weight to ≤ 20% by weight of water.

10. Process according to any one of Claims 1 to 8, wherein once discharged the bottom product from the first distillation unit (110, 1130) is diluted only with one composition comprising methanol.

11. Process according to any one of Claims 1 to 10, wherein the bottom product from the first distillation unit (110, 1130) is incinerated once it has been diluted.

## Revendications

1. Procédé de traitement d'un mélange de matières comprenant une amine aromatique, l'amine aromatique étant l'aniline ou le 2,4-diaminotoluène,
le mélange de matières étant un premier mélange de matières et comprenant une amine aromatique et des composés qui ont un point d'ébullition plus élevé que l'amine aromatique,
comprenant l'étape :
I) la séparation par distillation du premier mélange de matières dans une première unité de distillation (110, 1130) avec séparation au moins partielle de l'amine aromatique, un premier produit de fond étant également obtenu et ce premier produit de fond étant déchargé de la première unité de distillation (110, 1130) ;
**caractérisé en ce que**
le produit de fond de la première unité de distillation (110, 1130) est dilué après le déchargement avec un produit de tête condensé provenant d'une unité de distillation différente de la première unité de distillation et/ou avec une composition comprenant du méthanol.

2. Procédé selon la revendication 1, comprenant en outre les étapes Ia) et Ib), Ia) et Ib) étant réalisées avant I) :
Ia) la préparation d'un deuxième mélange de matières, le deuxième mélange de matières comprenant l'amine aromatique, des composés qui ont un point d'ébullition plus faible que l'amine aromatique et des composés qui ont un point d'ébullition plus élevé que l'amine aromatique, et dont la teneur en amine aromatique est différente de la teneur en amine aromatique du premier mélange de matières ;
Ib) la séparation par distillation du deuxième mélange de matières dans une unité de distillation en amont avec séparation de composés qui ont un point d'ébullition plus faible que l'amine aromatique en tant que produit de tête, l'amine aromatique étant en outre obtenue en tant que courant latéral et le premier mélange de matières étant obtenu en tant que produit de fond.

3. Procédé selon la revendication 1, comprenant en outre les étapes IIa), IIb) et III), IIa) et IIb) étant réalisées avant III), et III) avant I) :
IIa) la préparation d'un deuxième mélange de matières, le deuxième mélange de matières comprenant l'amine aromatique, des composés qui ont un point d'ébullition plus faible que l'amine aromatique et des composés qui ont un point d'ébullition plus élevé que l'amine aromatique, et dont la teneur en amine aromatique est différente de la teneur en amine aromatique du premier mélange de matières ;
IIb) la séparation par distillation du deuxième mélange de matières dans une deuxième unité de distillation (1110) avec séparation de composés qui ont un point d'ébullition plus faible que l'amine aromatique en tant que produit de tête, un produit de fond comprenant l'amine aromatique étant en outre obtenu ;
III) la séparation par distillation du produit de fond de la deuxième unité de distillation dans une troisième unité de distillation (1120) avec séparation au moins partielle de l'amine aromatique en tant que produit de tête, le premier mélange de matières étant en outre obtenu en tant que produit de fond.

4. Procédé selon l'une quelconque des revendications 1 à 3, dans lequel la teneur en amine aromatique du produit de fond de la première unité de distillation (110, 1130) est de ≥ 5 % en poids à ≤ 70 % en poids.

5. Procédé selon l'une quelconque des revendications 1 à 4, dans lequel le produit de fond de la première unité de distillation (110, 1130) est dilué après le déchargement avec le produit de tête provenant d'une unité de distillation différente de la première unité de distillation et/ou avec la composition comprenant du méthanol de sorte que le mélange obtenu présente une viscosité à 20 °C de ≥ 0,3 mPas à ≤ 1 000 mPas.

6. Procédé selon l'une quelconque des revendications 1 à 5, dans lequel la composition comprenant du méthanol est une composition comprenant du méthanol et de l'eau, qui a été obtenue à partir d'un procédé de fabrication de méthylène-diphényldiamine.

7. Procédé selon l'une quelconque des revendications 1 à 6, dans lequel la composition comprenant du méthanol contient de ≥ 20 % en poids à ≤ 95 % en poids de méthanol.

8. Procédé selon l'une quelconque des revendications 1 à 7, dans lequel le débit massique de la composition comprenant du méthanol est choisi de sorte que le débit massique calculé de méthanol pur soit ≥ 30 % en poids, par rapport au débit massique de produit de fond déchargé de la première unité de distillation (110, 1130).

9. Procédé selon l'une quelconque des revendications 1 à 8, dans lequel le produit de tête condensé issu d'une unité de distillation différente de la première unité de distillation contient de ≥ 80 % en poids à ≤ 100 % en poids de composés de point d'ébullition faible et ≥ 0 % en poids à ≤ 20 % en poids d'eau.

10. Procédé selon l'une quelconque des revendications 1 à 8, dans lequel le produit de fond de la première unité de distillation (110, 1130) n'est dilué après le déchargement qu'avec une composition comprenant du méthanol.

11. Procédé selon l'une quelconque des revendications 1 à 10, dans lequel le produit de fond de la première unité de distillation (110, 1130) est brûlé après la dilution.
